# EUROPÄISCHE PATENTSCHRIFT

(11) **EP 1 611 853 B1**
(45) Veröffentlichungstag und Bekanntmachung des Hinweises auf die Patenterteilung: **14.02.2007**
(21) Anmeldenummer: 05090197.4
(22) Anmeldetag: 29.06.2005
(51) Int. Cl.: A61B 17/122, A61B 17/128

(54) **Chirurgische Vorrichtung zum Abklemmen insbesondere von Blutgefässen**
Surgical tissue clamp device, in particular for blood vessels
Pince chirurgicale notamment pour les vaisseaux sanguins

(30) Priorität: 29.06.2004 DE 102004032450
(43) Veröffentlichungstag der Anmeldung: 04.01.2006
(73) Patentinhaber: Otten, Gert, 27619 Schiffdorf (DE)
(72) Erfinder: Otten, Gert, 27619 Schiffdorf (DE)
(74) Vertreter: Neumann, Günter

(56) Entgegenhaltungen:
- EP-A- 0 178 469

## Beschreibung

Die Erfindung betrifft eine Vorrichtung zum Abklemmen organischen Gewebes, insbesondere von Blutgefäßen nach dem Oberbegriff des Patentanspruch 1, anwendbar insbesondere in der Minimal Invasiven Chirurgie.

Eine derartige Vorrichtung ist bereits aus der EP 0 178 469 bekannt. Diese weist einen distalen und proximalen Klemmbalken auf, die sich senkrecht von einem Applikationssteg erstecken, wobei der proximale Klemmbalken in Richtung des distalen Klemmbalkens verschoben und zwischen ihnen das Gewebe abgeklemmt werden kann. Die so gebildete Gewebeklammer kann schließlich mittels eines den Applikationssteg umschließenden und zu diesem längs verschieblichen Stößels von dem Applikaionssteg entfernt werden, wozu dem Applikationssteg bzw. einer Basis des distalen Klemmbalkens eine Soll-Bruch-Stelle bzw. eine Ver- und Entriegelungseinrichtung zugeordnet ist. Die Nachteile dieser Vorrichtung sind insbesondere in ihrem komplizierten Aufbau und ihrer schwierigen Handhabung zu sehen. Vor allem das Bestücken des Applikationsstegs mit den Klemmbalken ist schwierig. Es setzt voraus, daß immer der proximale vor dem disalen Klemmbalken angeordnet wird. Außerdem dürfte eine sichere Fixierung der Klemmbalken zueinander in ihrer Endposition kaum möglich sein.

Der Erfindung liegt die Aufgabe zugrunde, eine Vorrichtung der eingangs erwähnten Art zu schaffen, die das exakte Erfassen und Abklemmen organischen Gewebes ermöglicht, ein Bestücken mit Klemmbalken variabel und in einfachster Weise gestattet, die Ausbildung der Gewebeklammer mit geringem Aufwand zuläßt und zudem kostengünstig einsetz- und herstellbar ist.

Erfindungsgemäß wird die Aufgabe dadurch gelöst, daß der Applikationssteg an seinem distalen Ende eine senkrecht zum Applikationssteg verlaufende erste Halterung aufweist, in die senkrecht zum Applikationssteg der erste Klemmbalken einschiebbar ist. Mit Abstand dazu in Richtung des Grundkörpers ist eine zweite Halterung vorgesehen, die parallel zur ersten verläuft und in die senkrecht zum Applikationssteg der zweite Klemmbalken einschiebbar ist. Beiden Halterungen sowie den zugehörigen Klemmbalken sind Halte- und Führungsmittel zugeordnet, die dem sicheren Einschieben und Fixieren der Klemmbalken dienen. Beide Halterungen sind ferner mittels einer in Längsrichtung des Applikationssteges verlaufenden Führungsnut miteinander verbunden, wobei entlang dieser Führungsnut mindestens einer der Klemmbalken zum anderen Klemmbalken verschoben werden kann. Zur exakten Verschiebung des Klemmbalkens innerhalb der Führungsnut ist dieser und dem zu verschiebenden Klemmbalken wenigstens eine Führungseinrichtung zugeordnet. Hierdurch ist es möglich, die Vorrichtung, d. h. dessen Applikationssteg in einfachster Weise mit den beiden Klemmbalken zu bestücken und die Gewebeklammer durch Zusammenfügen der Klemmbalken auszubilden. Schließlich ist den Klemmbalken eine Fixiereinrichtung zugeordnet, durch die eine exakte Annäherung der Klemmbalken unterstützt und deren Zusammenfügen zur Gewebeklammer ermöglicht wird.

Vorteilhaft ist es, daß das Einschieben bzw. Bestücken und Halten der Klemmbalken in die bzw. innerhalb der Halterungen des Applikationssteges kraft- und/oder formschlüssig erfolgen kann.

Mit geringem Aufwand erfolgt das Einschieben und Halten der Klemmbalken in die bzw. in den Halterungen überwiegend kraftschlüssig dadurch, daß die korrespondierenden Flächen des Applikationssteges und der Basen der Klemmbalken so diemensioniert sind, daß sie als Preßpassung wirken. Allein hierdurch kann zum einen der zweite, proximale Klemmbalken in der zugehörigen zweiten, proximalen Halterung solange sicher fixiert werden, bis dessen Verschiebung entlang des Applikationssteges mittels des Innenstößels eingeleitet wird. Zum anderen kann dadurch der distale, erste Klemmbalken in der distalen, ersten Halterung solange sicher fixiert werden, bis beide Klemmbalken in der distalen Halterung zu der Gewebeklammer zusammengefügt sind und mittels des Innenstößels von dem Applikationssteg herunter geschoben werden. Die lichte Weite der ersten distaen Halterung muß dabei stets gleich oder größer sein als die der zweiten proximalen Halterung, so daß der proximale Klemmbalken beim Herunterschieben der Gewebeklammer vom Applokationssteg auch die distale Halterung passieren kann.

Es ist ebenso möglich, daß das Einschieben und Halten der Klemmbalken zusätzlich bzw. ausschließlich formschlüssig erfolgt. Dabei sind den Halterungen und Klemmbalken Halte- und Führungsmittel zugeordnet, so daß eine exakte Positionierung der Klemmbalken in den Halterungen gewährleistet ist. Die Halte- und Führungsmittel sind durch mindestens eine sich innerhalb und senkrecht zum Applikationssteg erstreckende Quernut und wenigstens einen aus der Basis des zugehörigen Klemmbalkens vorstehenden korrespondierenden Vorsprung gebildet. Es kann aber auch sein, daß der Vorsprung der Halterung des Applikationssteges und die Quernut der Basis des Klemmbalkens zugeordnet ist.

In einfacher und zugleich sehr wirkungsvoller Weise kann die Position des distalen Klemmbalkens zum Applikationssteg dadurch gesichert werden, daß sich die Quernut der distalen Halterung in Einschubrichtung des ersten, distalen Klemmbalkens erstreckt und in dieser Quernut der Vorsprung des Klemmbalkens kraft- und formschlüssig eingreift, der als korrespondierende, von der Basis des ersten Klemmbalkens vorstehenden Fixierschulter ausgebildet ist.

Sowohl die Fixierung wie auch nachfolgende Trennung der Gewebeklammer von dem Applikationssteg wird dadurch begünstigt, daß die Fixierschulter elastisch ist und mit dem Erreichen bzw. Überschreiten der für die Trennung der Gewebeklammer von dem Applikationssteg erforderlichen Grenzkraft aus der Quernut austreten kann. Die Fixierschulter erstreckt sich vorzugsweise längs innerhalb der Quernut.

Die Positionierung des Klemmbalkens zum Applikationssteg kann zusätzlich dadurch begünstigt werden, daß die Quernut und Fixierschulter beidseitig der ersten Halterung bzw. Basis auf der dem Grundkörper nahen Seite angeordnet sind.

Die exakte Positionierung sowie Verschieblichkeit des zweiten Klemmbalkens kann mit einfachen Mitteln und hoher Funktionalität dadurch erreicht werden, daß als Halte- und Führungsmittel der zweiten Halterung mindestens eine sich in Einschubrichtung des zweiten Klemmbalkens erstreckende Quernut für den formschlüssigen Eingriff des korrespondierenden Vorsprungs, der als Führungsstift ausgebildet ist und von der Basis des zweiten Klemmbalkens vorsteht.

Die Positionierung und Verschieblichkeit kann ferner dadurch unterstützt werden, daß gegenüberliegend auf beiden Seiten der zweiten Halterung je eine der Quernuten vorgesehen ist, in die korrespondierend beidseitig der Basis des Klemmbalkens angeordnete Führungsstifte eingreifen. Dabei sind die Quernuten und Führungsstifte so dimensionert, daß sie nahezu spielfrei zusammenwirken und die senkrechte Position des Klemmbalkens zum Applikationssteg wie auch die Parallelität zueinander stets gegeben ist. Die Querschnitte der Quernuten und Führungsstifte müssen miteinander korrespondieren. Die der Führungsstifte sind vorzugsweise rund oder rechteckig und die der Quernut u-förmig.

Gemäß einer besonderen Ausführungsform ist vorgesehen, daß in Längsrichtung des Applikationsstegs hintereinander beidseitig jeweils zwei der Quernuten den Halterungen zugeordnet sind und in jede der Quernuten ein Paar der in Einschubrichtung der Klemmbalken hintereinander liegend korrespondierenden Führungsstifte eingreifen. Hierdurch greifen auf jeder Seite der Basis des Klemmbalkens zwei Paare von Führungsstiften in zwei Quernuten ein, wodurch insbesondere die exakte Positionierung des Klemmbalkens in der Halterung bzw. während dessen späteren Verschiebung nochmals unterstützt wird, sobald die beiden Paare der Führungsstifte dann in die Längsnuten der Führungsnut gelangen.

Die exakte Verschieblichkeit der Klemmbalken zueinander entlang des Applikationssteges wird in einfacher Weise dadurch ermöglicht, daß der die erste und zweite Halterung verbindende Führungsnut des Applikationssteges und dem zugehörigen Klemmbalken eine Führungseinrichtung zugeordnet ist. Diese ist durch mindestens eine Längsnut und wenigstens einen korrespondierenden Vorsprung bzw. Führungsstift gebildet, die wechselseitig entweder dem Applikationssteg oder dem Klemmbalken bzw. dessen Basis zugeordnet sind.

Vorzugsweise ist die Längsnut der Führungsnut des Applikationssteges und der korrespondierende Vorsprung bzw. Führungsstift dem jeweiligen Klemmbalken zugeordnet. Es kann aber auch vorteilhaft sein, daß die Führungsnut mit dem Vorsprung und der Klemmbalken mit der Längsnut versehen ist.

Die exakte Positionierung und Verschieblichkeit des Klemmbalkens kann zusätzlich dadurch verbessert werden, daß mehrere Längsnuten innerhalb der Führungsnut parallel nebeneinander und gegenüberliegend vorgesehen sind, in die entsprechend korrespondierend angeordnete Vorsprünge bzw. Führungsstifte des Klemmbalkens eingreifen. Der Querschnitt der Führungsnut des Applikationssteges kann demnach auch T- oder Doppel-T- förmige Gestalt aufweisen. Grundsätzlich gilt, daß die Halterungen, Klemmbalken und Führungsnut so dimensioniert sind, daß die aus den einzelnen Klemmbalken zusammen gesetzte Gewebeklammer aus der Führungsnut in Längsrichtung derselben heraus geschoben und von dem Applikationssteg getrennt werden kann. Die Breite der distalen Halterung muß also mindestens gleich oder größer der Breite der proximalen Halterung sein. Setzen sich die Längsnuten der Führungsnut des Applikationssteges nicht bis zum distalen Ende des Applikationssteges fort und ggf. lediglich bis zur distalen Halterung für den ersten Klemmbalken, was einer exakten Positionierung des distalen Klemmbalkens in der zugehörigen Halterung dienlich sein kann, muß der Führungsstift bzw. müssen die vorstehenden Führungsstifte des proximale Klemmbalkens und sofern bei diesem vorhanden auch die des distalen Klemmbalkens beim Herunterschieben der Gewebeklammer von dem Applikationssteg abgeschert werden. Dies erfolgt durch entsprechende Dimensionierung der Führungsstifte erst, nachdem die zum Abklemmen des Gewebes bzw. bleibenden Zusammenfügen der Klemmbalken erforderliche Kraftwirkung mittels des Innenstößels überschritten ist. Das beschriebene Abscheren der Führungsstifte kann aber auch vermieden bzw. ausgeschlossen werden, wenn die Längsnut bzw. Längsnuten der Führungsnut über die distale Halterung hinaus bis zum distalen Ende des Applikaionssteges geführt und in diesen die Führungsstifte aus dem Applikationssteg austreten können. Das Abscheren von Führungsstiften kann ferner dadurch ausgeschlossen werden, daß die lichte Weite der distalen Halterung und nachfolgenden Führungsnut so groß ist, daß diese vom proximalen Klemmbalken nebst vorstehenden Führungsstiften passiert werden können.

Die Quernuten der Halterungen und Längsnuten der Führungsnut münden ineinander, können sich aber auch kreuzen. Hierdurch ist es möglich, daß die Vorsprünge bzw. Führungsstifte der Basis des Klemmbalkens, der entlang des Applikationssteges verschieblich ist, zum Führung und Halten des Klemmbalkens sowohl in die Quernuten der Halterungen wie auch die Längsnuten der Führungsnut eingreifen bzw. überführt werden können.

Erfindungsgemäß ist ferner vorgesehen, daß die Fixiereinrichtung durch einen dem einen Klemmbalken zugeordneten Haltestift und eine dem anderen Klemmbalken zugeordnete koaxiale Haltebohrung gebildet ist, wobei diese in Längsrichtung des Applikationssteges verlaufen, so daß die Fixiereinrichtung in einfacher Weise ausgebildet werden kann.

Zweckmäßig ist es, daß den Basen der Klemmbalken der Haltestift bzw. die Haltebohrung zugeordnet und diese mit korrespondierendem Querschnitt ausgebildet sind, so daß die Fixiereinrichtung kaum mit dem abzuklemmenden Gewebe in Verbindung kommen kann. Dabei weisen der Haltestift und die Haltebohrung eine Überlappungslänge auf, die zum einen eine ausreichende Parallelität der Klemmbalken während des Abklemmens des Gewebes gewährleistet und zum anderen deren sichere gegenseitige Fixierung in der Endposition kraft- und/oder formschlüssig ermöglicht. Hierdurch ist eine hohe Funktionalität mit einfachen Mitteln erreichbar.

Es ist aber auch zweckmäßig, wenn die Klemmbalken nicht parallel sondern leicht konisch zueinander verlaufen, so daß beim Zusammenfügen der Klemmbalken zur Gewebeklammer zuerst die vom Applikationssteg entfernten Enden zur gegenseitigen Anlage kommen und durch entsprechende Verformung während des weiteren Zusammenfügens der Klemmbalken diese über eine größere Länge zur gegenseitigen Anlage kommen. Die Klemmbalken sind dabei gewissermaßen gegeneinander vorgespannt, was die Dimensionierung der auf das Gewebe einwirkenden Klemmkraft erleichtern kann.

In einer weiteren Ausgestaltung ist vorgesehen, daß der Querschnitt des Haltestifts und entsprechend der Haltebohrung die Form sich um einen Zentralpunkt gruppierender überlappender bzw. ineinander übergehender geometrischer Figuren aufweist, wodurch die zusammenwirkenden Oberflächen beider vergrößert und zusätzlich zum Kraftschluß auch einen Formschluß ermöglicht wird. Eine sichere Position der Klemmbalken zueinander kann dadurch wesentlich begünstigt werden.

In einfacher Weise ist der Querschnitt durch sternförmig um den Zentralpunkt angeordnete sich überlappende drei Kreise gebildet, so daß Längskanten entlang des Haltestifts gebildet sind, die eine Fixierung desselben in der Haltebohrung begünstigen.

Weitere Merkmale der Erfindung und deren Vorteile ergeben sich aus der übrigen Beschreibung sowie dem Patentanspruch.

Die Erfindung soll nachfolgend an einigen Ausführungsbeispielen näher erläutert werden. In der zugehörigen Zeichnung zeigen in schematischer und vergrößerter Darstellung:
- Fig. 1:: die Vorderansicht einer Vorrichtung, in teilweiser und teilweise geschnittener Darstellung,
- Fig. 2:: die Draufsicht eines Applikationsstegs nach Fig. 1, in teilweiser Darstellung,
- Fig. 3:: die Vorderansicht eines distalen Klemmbalkens nach Fig. 1,
- Fig. 4:: die Seitenansicht des Klemmbalkens nach Fig. 3,
- Fig. 5:: die Vorderansicht eines proximalen Klemmbalkens nach Fig. 1,
- Fig. 6:: die Draufsicht des Klemmbalkens nach Fig. 5,
- Fig.7:: die Vorderansicht eines weiteren Ausführungsbeispiels der Vorrichtung, in teilweiser und teilweise geschnittener Darstellung,
- Fig. 8:: die Draufsicht eines Applikationsstegs nach Fig. 7, in teilweiser Darstellung,
- Fig. 9:: die Vorderansicht eines distalen Klemmbalkens nach Fig. 7,
- Fig. 10:: die Seitenansicht des Klemmbalkens nach Fig. 9,
- Fig. 11:: die Draufsicht des Klemmbalkens nach Fig. 11,
- Fig. 12:: den Schnitt A - A nach Fig. 2 und
- Fig. 13:: den Schnitt quer durch ein weiteres Ausführungsbeispiel einer Vorrichtung.

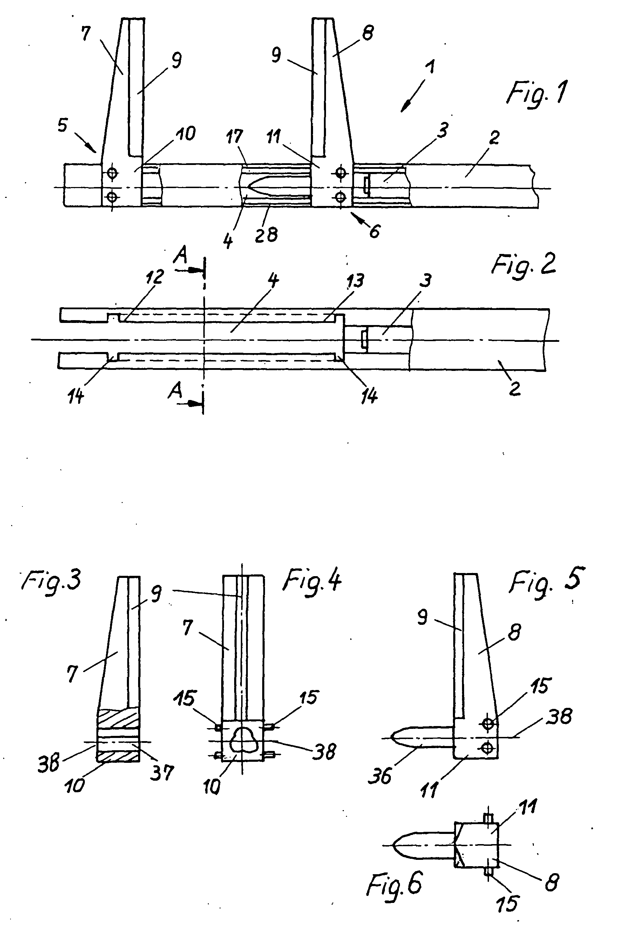

Die Vorrichtung 1 zum Abklemmen organischen Gewebes, insbesondere von Blutgefäßen (Fig. 1) weist einen Applikationssteg 2 auf, der sich von einem nicht näher beschriebenem proximalen Grundkörper in distaler Richtung erstreckt. Mit dem Grundkörper ist der Applikationssteg 2 an einem ebenfalls nicht näher beschriebenen Applikationsinstrument angeschlossen. Innerhalb des Applikationssteges 2 ist mittels des Applikationsinstruments ein Innenstößel 3 im wesentlichen über die gesamte Länge einer Führungsnut 4 des Applikationssteges 2 längsverschieblich, wobei sich die Führungsnut 4 auf der dem Applikationsinstrument abgewandten Seite von ihrem distalen Ende 5 bis zu ihrem proximalen Ende 6 erstreckt. Den Enden 5 und 6 ist jeweils ein Klemmbalken 7, 8 zugeordnet, zwischen deren Klemmstegen 9 das Blutgefäß abgeklemmt werden kann. Die Klemmbalken 7, 8 erstrecken sich von einer Basis 10, 11 derart, daß die Klemmstege 9 stets senkrecht zu dem Applikationssteg 2 sowie parallel zueinander angeordnet sind. Es ist aber auch möglich, daß die Klemmbalken 7, 8 bzw. deren Klemmstege 9 leicht konisch zueinander verlaufen und gewissermaßen gegeneinander vorgespannt sind, derart, daß beim Zusammenfügen der Klemmalken 7, 8 zuerst deren vom Applikationssteg 2 entfernten Bereiche zur gegenseitigen Anlage kommen und sich erst danach die übrigen Klemmstege 9 einander nähern.

Dem distalen Ende 5 der Führunsnut 4 ist eine Halterung 12 (Fig. 2) zugeordnet, in die senkrecht zum Applikationssteg 2 die Basis 10 des ersten, distalen Klemmbalkens 7 eingeschoben werden kann. Dem proximalen Ende 6 der Führungsnut 4 ist eine zweite, proximale Halterung 13 zugeordnet, in die senkrecht zum Applikationssteg 2 der zweite, proximale Klemmbalken 8 eingeschoben werden kann. Die Halterungen 12, 13 unterscheiden sich von der übrigen Führungsnut 4 dadurch, daß in ihnen die Klemmbalken 7, 8 auch fixiert werden können, während die Führungsnut 4 allein der Führung des proximalen Klemmbalkens 8 zum distalen Klemmbalken 7 dient. Wenngleich es ausreichen könnte, den Klemmbalken 7, 8 innerhalb der Halterung 12, 13 durch Preß- bzw. Übergangspassung der zusammenwirkenden Flächen der Führungsnut 4 und der Basis 10, 11 überwiegend durch Kraftschluß zu fixieren, sind den Halterungen 12, 13 und Basen 10, 11 Halte- und/oder Führungsmittel zugeordnet, die die sichere und exakte Positionierung zum einen während des Bestückens des Applikationssteges 2 wie auch zum anderen während der gegenseitigen Verschiebung der Klemmbalken 7, 8 allein oder zusätzlich durch Formschluß gewährleisten.

Die Halte- und Führungsmittel sind zum einen durch jeweils mindestens eine Quernut 14 (Fig. 2) der Halterung 12, 13 gebildet. Zum anderen ist mindestens ein zur Quernut 14 korrespondierender Vorsprung vorgesehen, der als Führungsstift 15 ausgebildet und dem Klemmbalken 7, 8 zugeordnet ist. Bei der Vorrichtung 1 sind auf jeder Seite der Basis 10, 11 zwei der Führungsstifte 15 vorgesehen (Fig. 1, 3, 4, 5, 6), die in Einschubrichtung der Klemmbalken 7, 8 hintereinander liegen, von der Basis 10, 11 vorstehen und nacheinander in die zugehörige Quernut 14 eingreifen, sobald der Klemmbalken 7, 8 in den Applikationssteg 2 eingeschoben bzw. der Applikationssteg 2 mit den Klemmbalken 7, 8 bestückt wird. Die Führungsstifte 15 und Quernuten 14 sind korrespondierend ausgebildet, derart, daß die Führungsstifte 15 in der Quernut 14 nahezu spielfrei geführt und gehalten werden, so daß die im wesentlichen senkrechte Erstreckung der Klemmbalken 7, 8 zum Applikationssteg 2 stets gegeben ist.

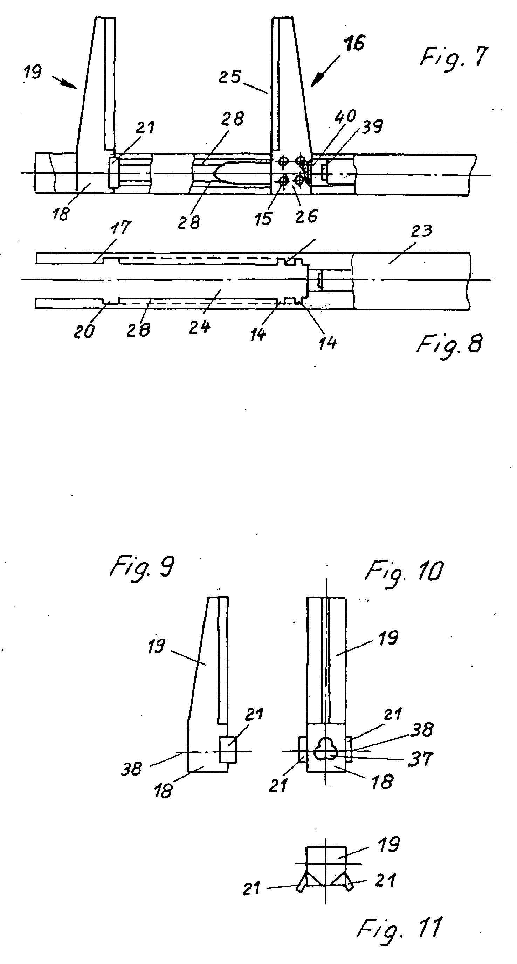

Bei einer Vorrichtung 16 gemäß Fig. 7 und 8 sind die Halte- und Führungsmittel einer distalen Halterung 17 eines Applkationssteges 23 und einer Basis 18 eines Klemmbalkens 19 zum einen durch eine Quernut 20 und zum anderen durch einen als vorstehende Fixierschulter 21 (Fig. 9, 10, 11) ausgebildeten Vorsprung gebildet. Die Fixierschulter 21 ist auf der proximalen Seite der Halterung 17 quer zum Applikatinssteg 23 angeordnet und greift über ihre gesamte Länge in die korrespondierende Quernut 20 ein. Sie ist elastisch und so dimensioniert, daß sie den distalen Klemmbalken 19 einerseits sicher in der Halterung 17 fixiert, andererseits aber aus der Quernut 20 austritt und den Klemmbalken 19 aus der Halterung 17 frei gibt, sobald das Zusammenfügen der Gewebeklammer mittels des Innenstößels 3 abgeschlossen ist und der Stößeldruck einen definierten Wert erreicht, bei dem die Gewebeklammer in Längsrichtung eines Applikationssteges 23 aus der Führungsnut 24 heraus geschoben und von dem Applikationssteg 23 getrennt wird. Ein proximaler Klemmbalken 25 der Vorrichtung 16 unterscheidet sich von dem zuvor beschriebenen dadurch, daß auf jeder Seite einer Basis 26 insgesamt vier der Führungsstifte 15 (Fig. 7) vorstehen, die paarweise in jeweils zwei parallele Quernuten 14 einer Halterung 27 eingreifen.

Die Halterungen 12, 13 bzw. 17, 27 sind mittel der Führungsnut 4 bzw. 24 miteinander verbunden, so daß der proximale Klemmbalken 8 bzw. 25 entlang der Führungsnut 4 bzw. 24 zum distalen Klemmbalken 7 bzw. 19 verschoben und beider zum Abklemmen eines Blutgefäßes sowie zur Ausbildung der Gewebeklammer verbunden werden können. Der Führungsnut 4, 24 und der Basis 11 bzw. 26 des Klemmbalkens 8 bzw. 25 sind wenigstens eine Führungseinrichtung zugeordnet, die der exakten Positionierung des Klemmbalkens 8, 25 während dessen Verschiebung entlang des Applikationssteges 2, 23 dient. Die Führungseinrichtung wird einerseits durch mindestens eine Längsnut 28, die sich entlang der Fühungsnut 4, 24 erstreckt, und andererseits durch wenigstens einen der sich von der Basis 11, 26 erhebenden Führungsstifte 15 gebildet. Beim Verschieben des Klemmbalkens 8, 25 entlang des Applikationssteges 2, 23 geht der Führungsstift 15 bzw. gehen die Führungsstifte 15 von der Quernut 14 in die Längsnut 28 über. Dies ist möglich, weil die Längsnut 28 in die Halterungen 12, 13, 20, 27 einmündet bzw. diese kreuzen.

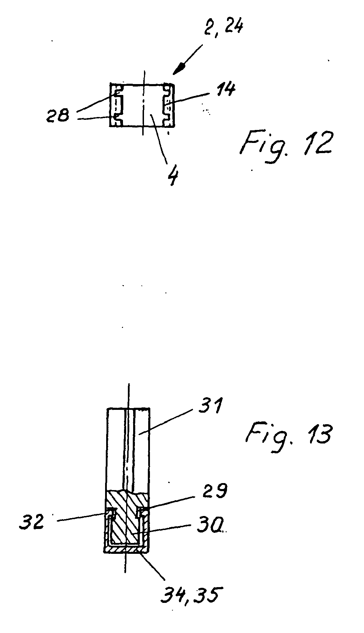

In jeder Seitenwand der Führungsnut 4, 24 können auch jeweils eine bzw. zwei oder mehrere parallele Längsnuten 28 angeordnet sein, so daß der Querschnitt der Führungsnut 4, 24 beispielsweise T- oder doppel-T- förmig ist. Bei doppel-T- förmigem Querschnitt (Fig. 12) kann die Positionierung des Klemmbalkens 8, 25 dadurch begünstigt werden, daß neben- bzw. übereinander auf jeder Seite der Basis 11, 26 sich zwei Paare der Führungsstifte 15 erheben und auch paarweise in die jeweilige Längsnut 28 eingreifen.

Gemäß dem speziellen Ausführungsbeispiel nach Fig. 13 kann die Führungseinrichtung auch durch wenigstens eine Längsnut 29 in einer Basis 30 eines Klemmbalkens 31 und mindestens einen vorsprungartigen Steg 32 gebildet sein, der sich entlang einer Führungsnut 33 oberhalb derselben in die Führungsnut 33 erstreckt. Hierbei ist ein Applikationssteg 34 nicht gabelförmig ausgebildet, sondern vorzugsweise mit einer Grundplatte 35 versehen. Gegen diese kann die Basis 29 beim Bestücken des Applikationssteges 34 und Einschieben mindestens eines proximalen Klemmbalkens 31 angelegt und so derart positioniert werden, daß der Steg 32 beim Verschieben des Klemmbalkens 31 entlang der Führungsnut 33 sicher in die Längsnut 29 eingreifen kann. Vorzugsweise werden gegenüberliegend sowohl die Längsnut 29 wie auch der Steg 32 paarweise angeordnet (Fig. 13). Gemäß einer weiteren Ausgestaltung ist es ferner möglich, daß die Führungsnut 33 auch zum Bestücken des Applikationssteges 34 mit dem proximalen Klemmbalken 31 genutzt und auf Aufnahmen wie die proximale Halterungen 13 verzichtet werden kann. In diesem Fall können beim Einschieben des Klemmbalkens 31 senkrecht zum Applikationssteg 34 in die Führungsnut 33 die oberen Bereiche des Applikationssteges 34 soweit auseinander gebogen werden, daß zuerst die Basis 29 zwischen die Stege gelangen und zum Ende des Bestückens der Steg 32 in die Längsnuten 29 einspringen und sodann der Klemmbalken 31 innerhalb der Führungsnut 33 verschoben werden kann. Dabei ist es schließlich auch möglich, den Steg 32, der ebenso als ein oder mehrere in Längsrichtung des Applikationssteges 34 hintereinander liegende Führungsstifte 15 ausgebildet sein kann, dem Klemmbalken 31 und die Führungsnut 33 dem Applikationssteg 34 zuzuordnen.

Während die Klemmbalken 7, 8, 19, 25, 31 in unterschiedlichster Weise ausgebildet und ihnen die verschiedensten korrespondierenden Halte- und Führungsmittel zugeordnet sein können, ist ihnen gemeinsam, daß den Klemmbalkens 7, 8, 19, 25, 31 jeweils paarweise eine Fixiereinrichtung zugeordnet ist, die im wesentlichen aus einem Haltestift 36 und einer korrespondierend ausgebildeten sowie koaxial angeordneten Haltebohrung 37 besteht. Diese Fixiereinrichtung dient einmal dazu, die jeweils zusammengehörenden beiden Klemmbalken 7, 8, 19,25 in der letzten Phase ihrer gegenseitigen Annäherung exakt zueinander zu positionieren und zum anderen deren dauerhafte Verbindung in ihre Endposition zu der Gewebekammer zu erreichen. Der Haltestift 36 und die Haltebohrung 37 sind den Basen 10, 11, 18, 26, 30 zugeordnet, so daß sie außerhalb des eigentlichen Klemmbereichs liegen und kaum mit dem abzuklemmenden Gewebe in Berührung kommen können. Die axiale Erstreckung derselbe bzw. die daraus resultierende und von der Dicke des abzuklemmenden Gewebes abhängige Überlappungslänge beider ist so dimensioniert, daß eine exakte Positionierung sowie dauerhafte Verbindung gegeben ist. Beide weisen korrespondierende Querschnitte auf, die ein kraft- und/oder formschlüssiges Zusammenwirken gestatten. Dies wird dadurch erreicht, daß deren Querschnitt durch sich um ihre gemeinsame Achse 38 sternförmig angeordnete geometrische Figuren, beispielsweise drei sich überlappende Kreise gebildet ist. Es ist aber auch jeder andere Querschnitt denkbar, sofern er den Kraft- und/oder Formschluß ermöglicht. Die Querschnitte des Haltestifts 36 und der Haltebohrung 37 können korrespondierend beispielsweise auch pyramiden-, kegel- oder ellipsenförmig sein.

Die distale Stirnseite des Innenstößels 3 und die gegenüberliegende proximale Stirnfläche der Basis 11, 26, des proximalen Klemmbalkens 8, 25 sind korrespondierend profiliert. Während der Innenstößel 3 einen Zentrierzapfen 39 aufweist ist die Basis 11, 26 mit einer korrespondierenden Zentrierbohrung 40 versehen, wobei auch umgekehrt der Zentrierzapfen 39 der Basis 11, 26 und die Zentrierbohrung 40 dem Innenstößel 3 zugeordnet sein kann. Durch das Ineinandergreifen der beiden kann die Führung bzw. Positionierung der proximalen Klemmbalken 8, 25 während deren Verschiebung entlang des Applikationssteges 2, 23 wesentlich begünstigt werden.

Das Abklemmen von Gewebe mittels der Vorrichtung 1, 16 erfolgt in folgender Weise. Der Applikationssteg 2, 23 wird mit den Klemmbalken 7, 8, 19, 25 bestückt, indem senkrecht zum Applikationssteg 2, 23 zuerst der distale Klemmbalken 7, 19 in die distale Halterung 12, 17 und danach der proximale Klemmbalken 8, 25 in die proximale Halterung 13, 27 eingeschoben werden. Dabei greifen die Führungsstift 15 bzw. die Fixierschulter 21 in die Quernuten 14 bzw. 20 ein. Anschließend wird der proximale Klemmbalken 8, 25 mittels des Innenstößels 3 soweit in Richtung des distalen Klemmbalkens 8, 19 verschoben, daß dessen Basis 11, 26 in die Führungsnut 4, 24 eintritt und die Führungsstifte 15 in die Längsnut 28 bzw. Längsnuten 28 der Führungsnut 4, 24 gelangen. Nachfolgend wird der mit den Klemmbalken 7, 8, 19, 25 bestückte Applikationssteg 2, 23 über einen nicht dargestellten Trokar zum Operationsfeld geführt. Dort wird das abzuklemmende Gewebe zwischen den Klemmbalken 7,8 bzw. 19, 25 erfaßt. Sodann wird der proximale Klemmbalken 8, 25 mittels des Innenstößels 3 weiter in Richtung des distalen Klemmbalkens 7, 19 verschoben, bis beide in ihrer Endposition zu der Gewebeklammer dauerhaft verbunden sind und das zwischen ihnen positionierte Gewebe abgeklemmt ist. Mit dem Erreichen der Endposition tritt der Haltestift 36 unter Ausildung einer kraft- und/oder formschlüssigen Verbindung in die Haltebohrung 37 ein. Schließlich wird die Gewebeklammer mittels des Innenstößels 3 von dem Applikationssteg 2, 23 herunter geschoben und von diesem getrennt. Dabei werden die von den Basen 10, 11, 18, 26 vorstehenden Führungsstifte 15 an der distalen Seite der distalen Halterung 12, 17 abgeschert bzw. tritt die Fixierschulter 21 aus der Quernut 20 aus, sobald die vom Innenstößel 3 ausgeübte Kraft den Wert übersteigt, der zum Zusammenfügen der Klemmbalken 7, 8, 19, 25 erforderlich ist. Der Applikationssteg 2, 23 kann anschließend vom Operationsfeld entfernt und aus dem Trokar gezogen werden.

Das Abscheren der vorstehenden Führungsstifte 15 erfolgt, weil die Längsnuten 28 in die distale Halterung 13, 18 einmündet und nicht bis zur distalen Stirnseite des Applikationsseges 2, 23 fortgeführt sind. In diesem Fall wird, wie beim Ausführungsbeispiel gemäß Fig. 1, die Fixierung des distalen Klemmbalkens 7 durch die vorstehenden Führungsstifte 15 wesentlich unterstützt. Durch entsprechende Dimensionierung der Führungsstifte 15 erfolgt das Abscheren erst, nachdem die zum Abklemmen des Gewebes bzw. bleibenden Zusammenfügen der Klemmbalken 7, 8 erforderliche Kraftwirkung mittels des Innenstößels 3 überschritten ist. Das beschriebene Abscheren der Führungsstifte 15 kann aber auch vermieden bzw. ausgeschlossen werden, wenn die Längsnut 28 bzw. Längsnuten 28 der Führungsnut 4 über die distale Halterung 12 hinaus bis zum distalen Ende 5 des Applikaionssteges 2 geführt und in diesen die Führungsstifte 15 aus dem Applikationssteg 2 austreten können. Das Abscheren von Führungsstiften 15 kann ferner dadurch ausgeschlossen werden, daß die lichte Weite der distalen Halterung 12 und nachfolgenden Führungsnut 4 so groß ist, daß diese vom proximalen Klemmbalken 8 nebst vorstehenden Führungsstiften 15 passiert werden kann.

Insbesondere bei dem Ausführungsbeispiel gemäß Fig. 7 und 8 können die Längsnuten 28 auch bis zur distalen Stirnseite des Applikainssteges 24 weitergeführt und so ein Abscheren der Führungsstifte 15 des proxialen Klemmbalkens 25 ausgeschlossen werde, erfolgt doch hier die sichere Fixierung des disalen Klemmbalkens 19 überwiegend durch die Fixierschulter 21 und zugehörige Quernut 20.

### Bezugszeichenaufstellung

- 1: Vorrichtung
- 2: Applikationssteg
- 3: Innenstößel
- 4: Führungsnut
- 5: Ende
- 6: Ende
- 7: Klemmbalken
- 8: Klemmbaken
- 9: Klemmsteg
- 10: Basis
- 11: Basis
- 12: Halterung
- 13: Halterung
- 14: Quernut
- 15: Führungsstift
- 16: Vorrichtung
- 17: Halterung
- 18: Basis
- 19: Klemmbalken
- 20: Quernut
- 21: Fixierschulter
- 22:
- 23: Applikationssteg
- 24: Führungsnut
- 25: Klemmbalken
- 26: Basis
- 27: Halterung
- 28: Längsnut
- 29: Längsnut
- 30: Basis
- 31: Klemmbalken
- 32: Steg
- 33: Führungsnut
- 34: Applikationssteg
- 35: Grundplatte
- 36: Haltestift
- 37: Haltebohrung
- 38: Achse
- 39: Zentrierzapfen
- 40: Zentrierbohrung

## Patentansprüche

1. Chirurgische Vorrichtung zum Abklemmen organischen Gewebes, insbesondere von Blutgefäßen mit einem Grundkörper, der lösbar am distalen Ende eines Applikationsinstrument anordenbar ist und von dem sich in distaler Richtung ein Applikationssteg (2;23) erstreckt, in dem ein Innenstößel (3) längsverschiebbar ist, wobei sich im wesentlichen senkrecht zum Applikationssteg erstreckende Klemmbalken (7,8;25,3) aus absorbierbarem Material mittels des Innenstößels von einer Ausgangsposition, in der sie einen großen gegenseitigen Abstand haben, in eine Endposition, in der sie einander nahe und zur Gewebeklammer zusammengefügt sind, zueinander verschiebbar sind, sowie in letzterer die Gewebeklammer von dem Applikationssteg trennbar ist, **dadurch gekennzeichnet, dass** der Applikationssteg (2;23) an seinem distalen Ende (5) eine erste Halterung (12;17) aufweist, in die senkrecht zum Applikationssteg (2;23) der erste Klemmbalken (7;19) einschiebbar ist und mit Abstand dazu an seinem proximalen Ende (6) eine zweite Halterung (13;27) vorgesehen ist, in die senkrecht zum Applikationssteg (2;23) der zweite Klemmbalken (8;25) einschiebbar ist, wobei beiden Halterungen (12;13;17;27) sowie den dazugehörigen Klemmbalken (7;8;19;25) Halte- und/oder Führungsmittel zugeordnet sind, die Halterungen (12;13;17;27) mittels einer in Längsrichtung des Applikationssteges (2;23) verlaufenden Führungsnut (4;24) miteinander verbunden sind, wobei der Führungsnut (4;24) und mindestens einem der Klemmbalken (7;8;19;25) wenigstens eine Führungseinrichtung zugeordnet ist, mittels der die gegenseitige Verschiebbarkeit der Klemmbalken (7;8;19;25) unter ständiger Beibehaltung der gegenseitigen Ausrichtung zueinander gegeben ist und die Klemmbalken (7;8;19;25) mit einer Fixiereinrichtung zur Fixierung derselben in ihrer Endposition versehen sind.

2. Vorrichtung nach Anspruch 1, **dadurch gekennzeichnet, dass** das Einschieben und Halten der Klemmbalken (7;8;19;25) in die bzw. innerhalb der Halterung (12;13;17;27) des Applikationssteges (2;23) kraft- und/oder formschlüssig erfolgt.

3. Vorrichtung nach Anspruch 1 oder 2, **dadurch gekennzeichnet, dass** das Einschieben und Halten der Klemmbalken (7;8;19;25) überwiegend kraftschlüssig erfolgt und die Halte- und/oder Führungsmittel durch Dimensionierung der miteinander korrespondierenden Flächen der Halterung (12;13;17;27) des Applikationssteges (2;23) und von Basen (10;11;18;26) der Klemmbalken (7;8;19;25) als Presspassung gebildet ist oder dass das Einschieben und Halten der Klemmbalken (7;8;19;25) überwiegend formschlüssig erfolgt und die den Halterungen (12;13;17;27) und Klemmbalken (7;8;19;25) zugeordneten Halte- und/oder Führungsmittel durch mindestens eine sich innerhalb und senkrecht zum Applikationssteg (2;23) erstreckende Quernut (14;20) und wenigstens einen aus einer Basis (10;11;18;26) des Klemmbalkens (7;8; 19;25) vorstehenden korrespondierenden Vorsprung gebildet sind.

4. Vorrichtung nach einem der Ansprüche 1 bis 3, **dadurch gekennzeichnet, dass** der Vorsprung der Halterung (12;13;17;27) des Applikationssteges (2;23) und die Quernut (14;20) der Basis (10;11;18;26) des Klemmbalkens (7;8;19;25) zugeordnet sind und/oder dass sich die Quernut (20) der distalen Halterung (17) in Einschubrichtung des ersten Klemmbalkens (19) erstreckt und in dieser der korrespondierende Vorsprung anordenbar ist, der als von der Basis (18) vorstehende Fixierschulter (21) ausgebildet ist.

5. Vorrichtung nach einem der Ansprüche 1 bis 4, **dadurch gekennzeichnet, dass** sich die Fixierschulter (21) längs in der Quernut (20) erstreckt und elastisch ist, derart, dass sie beim Verschieben des Klemmbalkens (19) zum Applikationssteg (23) und Erreichen der für die Trennung der Gewebeklammer von dem Applikationssteg (23) erforderlichen Grenzkraft aus der Quernut (20) heraustritt und die Gewebeklammer zum Applikationssteg (23) längsverschiebbar ist.

6. Vorrichtung nach einem der Ansprüche 1 bis 5, **dadurch gekennzeichnet, dass** die Quernut (20) und Fixierschulter (21) beidseitig der distalen ersten Halterung (17) auf der dem Grundkörper nahen Seite angeordnet sind und/oder dass als Halte- und Führungsmittel der Halterung (12;17;27) mindestens eine sich in Einschubrichtung des Klemmbalkens (7;8;25) erstreckende Quernut (14) für den formschlüssigen Eingriff des Vorsprungs, der als korrespondierender Führungsstift (15) aus der Basis (10;11;26) des Klemmbalkens (7;8;25) vorsteht.

7. Vorrichtung nach einem der Ansprüche 1 bis 6, **dadurch gekennzeichnet, dass** gegenüberliegend auf beiden Seiten der Halterung (12;17;27) je eine der Quernuten (14) vorgesehen sind, in die korrespondierend beidseitig der Basis (10;11;26) des Klemmbalkens (7;8;25) angeordnete Führungsstifte (15) eingreifen.

8. Vorrichtung nach einem der Ansprüche 1 bis 7, **dadurch gekennzeichnet, dass** in Längsrichtung des Applikationssteges (2;23) hintereinander beidseitig jeweils zwei der Quernuten (14) angeordnet sind und in jede der Führungsnuten (4;24) in Einschubrichtung der Klemmbalken (7;8;25) hintereinander liegend ein Paar von korrespondierenden Führungsstiften (15) eingreifen.

9. Vorrichtung nach einem der Ansprüche 1 bis 8, **dadurch gekennzeichnet, dass** die, die erste und zweite Halterung (12;13;17;27) verbindende Führungsnut (4;24), mit einer Führungseinrichtung zur Aufnahme und Führung des Klemmbalkens (8;25) während dessen Verschiebens entlang des Applikationssteges (2;23) versehen ist.

10. Vorrichtung nach einem der Ansprüche 1 bis 9, **dadurch gekennzeichnet, dass** die Führungseinrichtung durch wenigstens eine Längsnut (28) und mindestens einen hierzu korrespondierenden Vorsprung gebildet sind, die wechselseitig entweder dem Applikationssteg (2;23) oder Klemmbalken (8;25) zugeordnet sind.

11. Vorrichtung nach einem der Ansprüche 1 bis 10, **dadurch gekennzeichnet, dass** die Längsnut (28) in der Führungsnut (4;24) des Applikationssteges (2;23) verläuft und der korrespondierende Vorsprung von der Basis (11;26) des Klemmbalkens (8;25) vorsteht.

12. Vorrichtung nach einem der Ansprüche 1 bis 11, **dadurch gekennzeichnet, dass** mehrere der Längsnuten (28) vorgesehen sind, derart, dass der Querschnitt der Führungsnut (4;24) des Applikationssteges (2;23) eine T- oder Doppel-T-förmige Gestalt aufweist, in die korrespondierend beidseitig aus der Basis (11;26) des Klemmbalkens (8;25) vorstehende Vorsprünge eingreifen.

13. Vorrichtung nach einem der Ansprüche 1 bis 12, **dadurch gekennzeichnet, dass** mindestens ein Vorsprung von der Führungsnut (4;24) vorsteht und sich eine korrespondierende Längsnut (28) in der Basis (11;26) des Klemmbalkens (8;25) erstreckt.

14. Vorrichtung nach einem der Ansprüche 1 bis 13, **dadurch gekennzeichnet, dass** einem Applikationssteg (34) zugeordnete Führungsmittel durch mindestens einen Steg (32) und eine korrespondierende Längsnut (29) gebildet sind, die sich in Längsrichtung des Applikationssteges (34) erstrecken und alternativ entweder einer Führungsnut (33) des Applikationssteges (34) oder einer Basis (30) eines Klemmbalkens zugeordnet sind wobei sich vorzugsweise der Steg (32) vom Applikationssteg (34) quer in die Führungsnut (33) erstreckt und in die Längsnut (29) der Basis (30) eingreift.

15. Vorrichtung nach einem der Ansprüche 1 bis 14, **dadurch gekennzeichnet, dass** der Steg (32) und die Längsnut (29) paarweise auf beiden Seiten des Applikationssteges (34) bzw. der Basis (30) angeordnet sind und der Applikationssteg (34) zum Bestücken desselben mit dem Klemmbalken (31) aufweitbar ist, bis die Basis (39) in der Führungsnut (33) aufgenommen und der Steg (32) in die Längsnut (29) eingetreten ist.

16. Vorrichtung nach einem der Ansprüche 1 bis 15, **dadurch gekennzeichnet, dass** die Halterung (12;13), Klemmbalken (7;8;19;25), Quernut (14;20), Führungsnut (4;24) und Längsnut (28) derart dimensioniert sind, dass die Gewebeklammer mittels des Innenstößels (3) nach Erreichen der Endposition distal vom Applikationssteg (2;23) verschiebbar ist und/oder die Fixiereinrichtung durch einen Haltestift (36) und eine koaxiale Haltebohrung (37) gebildet ist, die sich in Längsrichtung des Applikationssteges (2;23) koaxial erstrecken und jeweils entweder dem einen oder anderen Klemmbalken (7;8;19;25) zugeordnet sind.

17. Vorrichtung nach einem der Ansprüche 1 bis 16, **dadurch gekennzeichnet, dass** der Haltestift (36) bzw. die Haltebohrung (37) den Basen (10;11;18;26) der Klemmbalken (7;8;19;25) zugeordnet sind, diese mit korrespondierendem Querschnitt ausgebildet sind und eine Überlappungslänge aufweisen, derart, dass zum einen die Parallelität der Klemmbalken (7;8;19;25) während des Abklemmens des Gewebes und zum anderen deren sichere gegenseitige Fixierung in der Endposition kraft- und/oder formschlüssig gegeben ist, wobei der Querschnitt vorzugsweise die Form sich um eine zentrale Achse (38) gruppierender überlappender bzw. ineinander übergehender geometrischer Figuren aufweist oder der Querschnitt durch sternförmig um die Achse (38) angeordnete sich überlappende Kreise gebildet ist.

18. Vorrichtung nach einem der Ansprüche 1 bis 17, **dadurch gekennzeichnet, dass** der Haltestift (36) und die Haltebohrung (37) korrespondierend zylindrisch, kegelförmig oder pyramidenförmig ausgebildet sind und/oder die Klemmbalken (7;8;19;25) leicht konisch bzw. vorgespannt zueinander verlaufen, so dass sie nahe des Applikationssteges (2;23) einen größeren gegenseitigen Abstand haben als entfernt von ihm.

19. Vorrichtung nach einem der Ansprüche 1 bis 18, **dadurch gekennzeichnet, dass** die distale Stirnfläche des Innenstößels (3) und die gegenüberliegende proximale Stirnfläche der Basis (11;26) des Klemmbalkens (8;25) mit korrespondierenden Profilierungen versehen sind wobei die Profilierungen vorzugsweise durch einen Zentrierzapfen (39) und eine Zentrierbohrung (40) gebildet sind, die entweder der Basis (11;26) oder dem Innenstößel (3) zugeordnet sind.

## Claims

1. Device for clamping organic tissue, in particular blood vessels with a base plate, that is attached in a removable manner to the distal end of an application instrument and from which an applicator base (2; 23) stretches out distally in which a inner pusher (3), whereby the clamping bars (7; 8;19;25) primarily vertical to the applicator base are of absorbing material and can be pushed toward one another from the initial position, where they have a large distance from one another, to the final position, where they are close to one another and, **characterized by**, the applicator base (2; 23) has a first holder (12; 17) on its distal end (5), wherein perpendicular to the applicator base (2; 23) the first clamp bar (7; 19) can be inserted and at a distance from this the second clamp bar (8; 25) can be inserted perpendicularly to the applicator base (2; 23), whereby the two holders (12; 13; 17; 27) as well as the corresponding clamp bars (7; 8; 19; 25) have holders and/or guides which connect the holders (12; 13; 17; 27) by means of a guide slot (4; 24) running the length of the applicator base (2; 23), whereby the guide slot (4; 24) and at least one of the 7xa has at least guide device by means of which the mutual mobility of the clamp bars (7; 8; 19; 25) is assured while continuously maintaining the opposite orientation to one another and the clamp bars (7; 8; 19; 25) have an attachment device to attach themselves in their final position.

2. Device in accordance with claim 1, **characterized by** the inserting and holding of the clamp bars (7; 8; 19; 25) into or within the holders (12; 13; 17; 27) of the applicator base (2; 23) is force- and form-fitting.

3. Device in accordance with one of the claims 1 or 2, **characterized by** the inserting and holding of the clamp bars (7; 8; 19; 25) primarily being force-fitting and the holding and/or guiding is formed by the dimensioning of the corresponding areas of the holders (12; 13; 17; 27) of the applicator base (2; 23) and the bases (10; 11; 18; 26) of the clamp bars (7; 8; 19; 25) as a press fit and/or the inserting and holding of the clamp bars (7; 8; 19; 25) primarily being force-fitting and the holding and/or guiding of the holders (12; 13; 17; 27) and the clamp bars (7; 8; 19; 25) is formed by at least one traverse slot (14; 20) within and perpendicular to the applicator base (2; 23) and at least one corresponding projection rising from a base (10; 11; 18; 26) of the clamp bars (7; 8; 19; 25).

4. Device in accordance with one of the claims 1 to 3, **characterized by** projection of the holders (12; 13; 17; 27) are located on the applicator base (2; 23) and the traverse slot (14; 20) on the bases (10; 11; 18; 26) of the clamp bars (7; 8; 19; 25) and/or the traverse slot (20) of the distal holder (17) is in the direction of the fist clamp bar (19) and a corresponding projection is on this which is formed as a projecting attachment shoulder (21) from the base (18).

5. Device in accordance with one of the claims 1 to 4, **characterized by** an attachment shoulder (21) runs along the traverse slot (20) and is elastic in such a way that when moving the clamp bar (19) toward the applicator base (23) and that it exits from the traverse slot (20) when the force limit for the separation of the tissue clamp of the applicator base (23) and the tissue clamp can be moved along the applicator base (23).

6. Device in accordance with one of the claims 1 to 5, **characterized by** the traverse slot (20) and the attachment shoulder (21) being on both sides of the distal first holder (17) on the side closer to the base at least one traverse slot (14) in the insertion direction of the clamp bars (7; 8; 25) projects as holder and guide of the holders (12; 17; 27) for the form-fit insertion in the projection which projects as corresponding guide pin (15) from the bases (10; 11; 26) of the clamp bars (7; 8; 25).

7. Device in accordance with one of the claims 1 to 6, **characterized by** one of the traverse slots (14) is foreseen on each side of the holders (12; 17; 27) which can slip into the corresponding guide pins (15) on either side of the bases (10; 11; 26) of the clamp bars (7; 8; 25).

8. Device in accordance with one of the claims 1 to 7, **characterized by** two of the traverse slots (14) are located one behind the other on both sides in the longitudinal direction of the applicator base (2; 23) and in each of the guide slots (4; 24) a pair of corresponding guide pins (15) slip into the insertion direction of the clamp bars (7; 8; 25) one behind the other.

9. Device in accordance with one of the claims 1 to 8, **characterized by** the guide slot (4; 24) connecting the first and second holders (12; 13; 17; 27) have a guiding device to take up and guide the clamp bar (8; 25) while it is moving along the applicator base (2; 23).

10. Device in accordance with one of the claims 1 to 9, **characterized by** the guide device having at least one longitudinal slot (28) and at least one corresponding projection to this is formed that is located such that it is either for the applicator base (2; 23) or the clamp bar (8; 25).

11. Device in accordance with one of the claims 1 to 10, **characterized by** the longitudinal slot (28) being in the guide slot (4; 24) of the applicator base (2; 23) and the corresponding projection projects from the base (11; 26) of the clamp bar (8; 25).

12. Device in accordance with one of the claims 1 to 11, **characterized by** there being several longitudinal slots (28) of which the cross-section of the guide slot (4; 24) of the applicator base (2; 23) having a T or double T-shaped form into which the corresponding projections from the base (11; 26) of the clamp bar (8; 25) slip.

13. Device in accordance with one of the claims 1 to 12, **characterized by** at least one projection projects from the guide slot (4; 24) and a corresponding longitudinal slot (28) stretches along the base (11; 26) of the clamp bar (8; 25).

14. Device in accordance with one of the claims 1 to 13, **characterized by** a guide in an applicator base (34) is formed by at least one base (32) and a corresponding longitudinal slot (29) which stretches along the applicator base (34) and is allocated to either a guide slot (33) of the applicator base (34) or a base of a clamp bar (30), whereby the base (32) of the applicator base (34) stretches into the guide slot (33) and slips into the longitudinal slot (29) of the base (30).

15. Device in accordance with one of the claims 1 to 14, **characterized by** the base (32) and the longitudinal slot (29) located together on both sides of the applicator base (34) or base (30) and the applicator base (34) can be spread out to equip it with the clamp bar (31) until the base (30) is taken up in the guide slot (33) and the base (32) enters into the longitudinal slot (29).

16. Device in accordance with one of the claims 1 to 15, **characterized by** the holder (12; 13), the clamp bars (7; 8; 19; 25), traverse slot (14; 20), guide slot (4; 24) and longitudinal slot (28) dimensioned so that the tissue clamp can be pushed distally from the applicator base (2; 23) by means of the inner push (3) after reaching the final position and/or the attachment device being formed by a holding pin (36) and coaxial bore (37) that runs coaxially the length of the applicator base (2; 23) and is on either the one or the other clamp bars (7; 8; 19; 25).

17. Device in accordance with one of the claims 1 to 16, **characterized by** the holding pin (36) and the holding bore (37) is located on the bases (10; 11; 18; 26) of the clamp bars (7; 8; 19; 25), these formed with corresponding cross-section and an overlapping of such that they are parallel to the clamp bars (7; 8; 19; 25) during the clamping of the tissue and also they are securely fastened in the final position both force and form-fitting, whereby the cross-section shows the form of geometric figures crossing over and merging with one another around a central axis (38) or the cross-section being formed by overlapping circles forming a star around an axis (38).

18. Device in accordance with one of the claims 1 to 17, **characterized by** the holding pin (36) and the holding bore (37) being shaped correspondingly either as pyramids, cones, or cylinders and/or the clamp bars (7; 8; 19; 25) being slightly conical and pre-stressed toward one another to that they have a greater distance to one another near the applicator base (2; 23) than they do farther away from it.

19. Device in accordance with one of the claims 1 to 18, **characterized by** the distal front surface of the inner push (3) and the proximal front surface of the base (11; 26) of the clamp bar (8; 25) have corresponding profiles, whereby the profiles are shaped by a centering pin (39) and a centering bore (40) that is located either on the base (11; 26) or the inner push (3).

## Revendications

1. Dispositif chirurgical pour pincer le tissu organique, notamment les vaisseaux sanguins, composé d'un corps de base qui peut être disposé de façon amovible à l'extrémité distale d'un instrument d'application et duquel part dans le sens distal un applicateur (2;23), dans lequel un poussoir intérieur (3) peut se déplacer longitudinalement, des barres de serrage (7;8;19;25) qui sont sensiblement perpendiculaires à l'applicateur et se composent d'un matériau pouvant être absorbé peuvent se déplacer l'une par rapport à l'autre au moyen du poussoir intérieur d'une position de départ, dans laquelle elles sont très écartées l'une de l'autre, dans une position terminale, dans laquelle elles se rapprochent l'une de l'autre ainsi que vers la pince à serrer le tissu, la pince à serrer le tissu pouvant être séparée de l'applicateur dans cette dernière position, **caractérisé en ce que** l'applicateur (2;23) présente à son extrémité distale (5) une première fixation (12;17) dans laquelle la première barre de serrage (7;19) peut être rentrée perpendiculairement à l'applicateur (2;23), et une seconde fixation (13;27) dans laquelle la seconde barre de serrage (8;25) peut être rentrée perpendiculairement à l'applicateur (2;23) est prévue à son extrémité proximale (6) en étant écartée de la première, des moyens de maintien et/ou de guidage étant associés aux deux fixations (12;13;17;27) ainsi qu'aux barres de serrage associées (7;8;19;25), les fixations (12;13;17;27) sont reliées ensemble au moyen d'une rainure de guidage (4;24) s'étendant dans le sens longitudinal de l'applicateur (2;23), au moins un dispositif de guidage étant associé à la rainure de guidage (4;24) et au moins à l'une des barres de serrage (7;8;19;25), dispositif de guidage permettant de déplacer les barres de serrage (7;8;19;25) l'une par rapport à l'autre en conservant constamment l'orientation de celles-ci l'une par rapport à l'autre, et les barres de serrage (7;8;19;25) sont pourvues d'un dispositif de fixation destiné à fixer celles-ci dans leur position terminale.

2. Dispositif selon la revendication 1, **caractérisé en ce que** le glissement et le maintien des barres de serrage (7;8;19;25) dans ou à l'intérieur de la fixation (12;13;17;27) de l'applicateur (2;23) sont réalisés grâce aux forces exercées et/ou à la complémentarité des formes.

3. Dispositif selon la revendication 1 ou 2, **caractérisé en ce que** le glissement et le maintien des barres de serrage (7;8;19;25) sont réalisés principalement grâce aux forces exercées par le fait que les moyens de maintien et/ou de guidage se présentent sous forme d'ajustage serré dû au dimensionnement des surfaces correspondant l'une avec l'autre de la fixation (12;13;17;27) de l'applicateur (2;23) et des bases (10; 11;18;26) des barres de serrage (7;8;19;25), ou que le glissement et le maintien des barres de serrage (7;8;19;25) sont réalisés principalement grâce à la complémentarité des formes par le fait que les moyens de maintien et/ou de guidage associés aux fixations (12;13;17;27) et aux barres de serrage (7;8;19;25) sont formés par au moins une rainure transversale (14;20) s'étendant à l'intérieur de l'applicateur (2;23) et perpendiculairement à celui-ci et par au moins une saillie correspondante dépassant de la base (10; 11;18;26) de la barre de serrage (7;8; 19;25).

4. Dispositif selon l'une quelconque des revendications 1 à 3, **caractérisé en ce que** la saillie est associée à la fixation (12;13;17;27) de l'applicateur (2;23) et la rainure transversale (14;20) est associée à la base (10;11;18;26) de la barre de serrage (7;8;19;25) et/ou que la rainure transversale (20) de la fixation distale (17) s'étire dans le sens de glissement de la première barre de serrage (19) et la saillie correspondante réalisée en épaule de fixation (21) dépassant de la base (18) peut être disposée à l'intérieur de celle-ci.

5. Dispositif selon l'une quelconque des revendications 1 à 4, **caractérisé en ce que** l'épaule de fixation (21) s'étire longitudinalement dans la rainure transversale (20) et est élastique de sorte que, lors du déplacement de la barre de serrage (19) vers l'applicateur (23) et en atteignant la force limite nécessaire pour séparer la pince à serrer le tissu de l'applicateur (23), elle sort de la rainure transversale (20), la pince à serrer le tissu pouvant se déplacer longitudinalement par rapport à l'applicateur (23).

6. Dispositif selon l'une quelconque des revendications 1 à 5, **caractérisé en ce que** la rainure transversale (20) et l'épaule de fixation (21) sont disposées des deux côtés de la première fixation (17) distale sur la face proche du corps de base et/ou qu'au moins une rainure transversale (14) s'étirant dans le sens de glissement de la barre de serrage (7;8;25) et dans laquelle vient s'engager la saillie de forme complémentaire, dépassant sous forme de tige de guidage correspondante (15) de la base (10;11;26) de la barre de serrage (7;8;25), est réalisée sous forme de moyens de maintien et de guidage de la fixation (12;17;27).

7. Dispositif selon l'une quelconque des revendications 1 à 6, **caractérisé en ce que** sur chacun des deux côtés de la fixation (12;17;27) se faisant face, il est prévu une rainure transversale (14) dans laquelle viennent s'engager des tiges de guidage (15) disposées de façon correspondante des deux côtés de la base (10;11;26) de la barre de serrage (7;8;25).

8. Dispositif selon l'une quelconque des revendications 1 à 7, **caractérisé en ce que**, dans le sens longitudinal de l'applicateur (2;23), deux rainures transversales (14) sont disposées à chaque fois l'une derrière l'autre des deux côtés et une paire de tiges de guidage correspondantes (15), placées l'une derrière l'autre, vient s'engager dans chacune des rainures de guidage (4;24) dans le sens de glissement des barres de serrage (7;8;25).

9. Dispositif selon l'une quelconque des revendications 1 à 8, **caractérisé en ce que** la rainure de guidage (4;24) reliant la première et la seconde fixation (12;13;17;27) est pourvue d'un dispositif de guidage destiné à loger et guider la barre de serrage (8;25) pendant son déplacement le long de l'applicateur (2;23).

10. Dispositif selon l'une quelconque des revendications 1 à 9, **caractérisé en ce que** le dispositif de guidage est formé par au moins une rainure longitudinale (28) et au moins une saillie lui correspondant qui sont associées en alternance soit à l'applicateur (2;23) soit aux barres de serrage (8;25).

11. Dispositif selon l'une quelconque des revendications 1 à 10, **caractérisé en ce que** la rainure longitudinale (28) s'étend dans la rainure de guidage (4;24) de l'applicateur (2;23) et la saillie correspondante dépasse de la base (11;26) de la barre de serrage (8;25).

12. Dispositif selon l'une quelconque des revendications 1 à 11, **caractérisé en ce qu'**il est prévu plusieurs rainures longitudinales (28) de sorte que la section transversale de la rainure de guidage (4;24) de l'applicateur (2;23) présente une forme en T ou en double T dans laquelle viennent s'engager des saillies correspondantes dépassant des deux côtés de la base (11;26) de la barre de serrage (8;25).

13. Dispositif selon l'une quelconque des revendications 1 à 12, **caractérisé en ce qu'**au moins une saillie dépasse de la rainure de guidage (4;24) et une rainure longitudinale correspondante (28) s'étend dans la base (11;26) de la barre de serrage (8;25).

14. Dispositif selon l'une quelconque des revendications 1 à 13, **caractérisé en ce que** des moyens de guidage associés à l'applicateur (34) sont formés par au moins une ailette (32) et une rainure longitudinale correspondante (29) qui s'étendent dans le sens longitudinal de l'applicateur (34) et sont associées au choix soit à une rainure de guidage (33) de l'applicateur (34) soit à la base (30) d'une barre de serrage, l'ailette (32) s'étendant de préférence depuis l'applicateur (34) diagonalement dans la rainure de guidage (33) pour venir s'engager dans la rainure longitudinale (29) de la base (30).

15. Dispositif selon l'une des revendications 1 à 14, **caractérisé en ce que** l'ailette (32) et la rainure longitudinale (29) sont disposées par paire des deux côtés de l'applicateur (34) et de la base (30) respectivement et l'applicateur (34) peut être élargi pour recevoir la barre de serrage (31) jusqu'à ce que la base (39) se loge dans la rainure de guidage (33) et l'ailette (32) soit rentrée dans la rainure longitudinale (29).

16. Dispositif selon l'une quelconque des revendications 1 à 15, **caractérisé en ce que** la fixation (12;13), les barres de serrage (7;8;19;25), la rainure transversale (14;20), la rainure de guidage (4;24) et la rainure longitudinale (28) sont dimensionnées de sorte que la pince à serrer le tissu peut se déplacer au moyen du poussoir intérieur (3) dans le sens distal par rapport à l'applicateur (2;23) après avoir atteint sa position terminale et/ou le dispositif de fixation est formé par une tige de maintien (36) et un perçage de maintien coaxial (37) qui s'étendent coaxialement dans le sens longitudinal de l'applicateur (2;23) et sont associés à chaque fois soit à l'une ou l'autre des barres de serrage (7;8;19;25).

17. Dispositif selon l'une quelconque des revendications 1 à 16, **caractérisé en ce que** la tige de maintien (36) et le perçage de maintien (37) respectivement sont associés aux bases (10;11;18;26) des barres de serrage (7;8;19;25), ceux-ci présentant une section transversale correspondante ainsi qu'une longueur de recouvrement telles que, d'une part, le parallélisme des barres de serrage (7;8;19;25) pendant le serrage du tissu et, d'autre part, leur fixation réciproque sûre sont assurés en position terminale grâce aux forces exercées et/ou à la complémentarité des formes, la section transversale présentant de préférence la forme de figures géométriques se chevauchant en se regroupant autour d'un axe central (38) ou passant l'une dans l'autre, ou la section transversale est formée par des cercles se chevauchant, disposés en forme d'étoile autour de l'axe (38).

18. Dispositif selon l'une quelconque des revendications 1 à 17, **caractérisé en ce que** la tige de maintien (36) et le perçage de maintien (37) présentent une forme correspondante cylindrique, conique ou pyramidale et/ou les barres de serrage (7;8;19;25) convergent l'une vers l'autre de façon légèrement conique ou en étant prétendues de sorte que leur écart réciproque est plus important lorsqu'elles sont proches de l'applicateur (2;23) que lorsqu'elles en sont éloignées.

19. Dispositif selon l'une quelconque des revendications 1 à 18, **caractérisé en ce que** la face frontale distale du poussoir intérieur (3) et la face frontale proximale opposée de la base (11;26) de la barre de serrage (8;25) sont pourvues de profilages correspondants, lesdits profilages étant formés de préférence par une broche de centrage (39) et un perçage de centrage (40) qui sont associés soit à la base (11;26), soit au poussoir intérieur (3).
